# FASCICULE DE BREVET EUROPEEN

(11) **EP 1 867 351 B1**
(45) Date de publication et mention de la délivrance du brevet: **24.09.2008**
(21) Numéro de dépôt: 07290724.9
(22) Date de dépôt: 11.06.2007
(51) Int. Cl.: A61M 1/10, A61M 1/12

(54) **Dispositif de raccordement entre une prothèse cardiaque et les oreillettes naturelles**
Verbindungsvorrichtung zwischen einer Herzprothese und den natürlichen Herzvorhöfen
Connection device between an artificial heart and the natural atria

(30) Priorité: 15.06.2006 FR 0605332
(43) Date de publication de la demande: 19.12.2007
(73) Titulaire: Groupement Coeur Artificiel Total Carpentier Matra Carmat, 78140 Velizy Villacoublay (FR)
(72) Inventeur: Parquet, Jean-Marc, 95330 Domont (FR); Carpentier, Alain, 75116 Paris (FR); Bareau, Pascal, 91470 Limours (FR); Capel, Antoine, 92140 Clamart (FR)
(74) Mandataire: Bonnetat, Christian

(56) Documents cités:
- EP-A1- 0 324 669
- FR-A- 2 107 724
- FR-A1- 2 784 585

## Description

La présente invention concerne un dispositif de raccordement entre une prothèse cardiaque et les oreillettes naturelles.

Par le document US-5 135 539, on connaît déjà une prothèse cardiaque, implantable dans la cavité péricardique d'un patient et apte à remplacer les ventricules gauche et droit naturels dudit patient, après ablation de ceux-ci. Cette prothèse cardiaque comporte un corps rigide dans lequel sont agencés des ventricules gauche et droit artificiels, lesdits ventricules artificiels étant pourvus de moyens de raccord rapide aux oreillettes naturelles gauche et droite dudit patient comportant :
- une première lunette, faisant partie intégrante dudit corps rigide et comportant des premier et deuxième orifices, respectivement en communication avec le ventricule artificiel gauche et avec le ventricule artificiel droit, par l'intermédiaire de valves ; et
- une seconde lunette comportant des troisième et quatrième orifices aptes à être respectivement reliés à ladite oreillette naturelle gauche et à ladite oreillette naturelle droite.

Dans cette réalisation, lesdites première et seconde lunettes sont aptes à être raccordées l'une à l'autre, de façon amovible, pour prendre une position de fonctionnement dans laquelle se trouvent respectivement en regard, d'une part, lesdits premier et troisième orifices et, d'autre part, lesdits deuxième et quatrième orifices.

Un tel système de lunettes facilite grandement le raccordement de ladite prothèse à ladite seconde lunette, préalablement raccordée auxdites oreillettes naturelles.

Pour relier ladite seconde lunette aux oreillettes naturelles, le document US-5 135 539 prévoit de munir lesdits troisième et quatrième orifices d'un anneau de suture sur lequel sont respectivement suturées, de façon étanche, lesdites oreillettes. Or, du fait que lesdites oreillettes, d'une part, sont constituées de tissus souples aisément déformables, et, d'autre part, ne sont pas totalement indépendantes, car reliées par le septum inter-auriculaire, il peut se faire que, après suture, lesdites oreillettes ne présentent pas, par rapport à ladite seconde lunette, l'orientation optimale leur évitant torsion et compression. Dans ce cas, de tels défauts d'orientation ne peuvent être corrigés que par de nouvelles sutures après désolidarisation des oreillettes dudit anneau de suture.

La présente invention a pour objet de remédier à cet inconvénient.

A cette fin, selon l'invention, la prothèse cardiaque implantable dans la cavité péricardique d'un patient, ladite prothèse étant apte à remplacer les ventricules gauche et droit naturels dudit patient après ablation de ceux-ci et comportant un corps rigide dans lequel sont agencés des ventricules artificiels gauche et droit, lesdits ventricules artificiels étant pourvus de moyens de raccord aux oreillettes gauche et droite naturelles dudit patient comportant :
- une première lunette, faisant partie intégrante dudit corps rigide et comportant des premier et deuxième orifices, respectivement en communication avec le ventricule artificiel gauche et avec le ventricule artificiel droit ; et
- une seconde lunette comportant des troisième et quatrième orifices aptes à être respectivement reliés à ladite oreillette naturelle gauche et à ladite oreillette naturelle droite, lesdits troisième et quatrième orifices étant pourvus de moyens de jonction sur lesquels lesdites oreillettes naturelles sont respectivement suturées de façon étanche,
lesdites première et seconde lunettes étant aptes à être raccordées l'une à l'autre, de façon amovible, pour prendre une position de fonctionnement dans laquelle se trouvent respectivement en regard, d'une part, lesdits premier et troisième orifices et, d'autre part, lesdits deuxième et quatrième orifices,
est remarquable en ce que lesdits moyens de jonction sont formés par des collerettes individuelles de suture montées de façon à être libres en rotation, respectivement sur lesdits troisième et quatrième orifices.

Ainsi, il est possible de corriger des erreurs d'orientation des oreillettes par simple rotation des collerettes par rapport à ladite seconde lunette.

De plus, il est avantageux que lesdites collerettes de suture individuelles soient montées de façon amovible sur lesdits troisième et quatrième orifices, respectivement.

Ainsi, lesdites collerettes de suture individuelles étant amovibles par rapport à la seconde lunette, il en résulte que, éventuellement, la suture d'une oreillette sur une collerette peut être réalisée lorsque la collerette est momentanément séparée de la seconde lunette, ce qui, parfois, peut simplifier l'opération.

Dans un premier exemple de l'invention, chaque collerette de suture individuelle comporte une ouverture centrale délimitée par un bord périphérique interne et ladite seconde lunette comporte, autour de chacun desdits troisième et quatrième orifices, une saillie annulaire formant une gorge à l'intérieur de laquelle est engagé ledit bord de ladite ouverture centrale.

En variante, chaque collerette de suture individuelle comporte une ouverture centrale délimitée par un rebord apte à enserrer le bord du troisième ou du quatrième orifice, respectivement. Dans ce cas, il est avantageux que ledit rebord de la collerette de suture comporte un prolongement périphérique s'appliquant contre la face interne de ladite seconde lunette. Ainsi, le sang contenu dans l'oreillette n'a comme interface qu'un matériau unique. Il est également avantageux que ce prolongement comporte, à son extrémité, un anneau de matière élastique, par exemple un élastomère, pouvant servir de joint d'étanchéité lorsqu'il est pressé entre lesdites lunettes en position raccordée et contribuant au positionnement axial de ladite collerette par rapport au troisième ou quatrième orifice, respectivement. A cet effet, la face interne de la seconde lunette peut comporter une saillie annulaire, qui entoure ledit troisième ou quatrième orifice et derrière laquelle peut s'accrocher élastiquement ledit anneau élastique.

Les figures du dessin annexé feront bien comprendre comment l'invention peut être réalisée. Sur ces figures, des références identiques désignent des éléments semblables.

La figure 1 est une vue schématique en perspective éclatée d'une prothèse cardiaque conforme à la présente invention.

La figure 2 est une vue partielle, en perspective du dessus, de la prothèse cardiaque de la figure 1, montée sur la lunette de raccord aux oreillettes naturelles.

Les figures 3 et 4 illustrent, en coupe diamétrale à plus grande échelle, deux modes de réalisation de collerettes de suture desdites oreillettes naturelles.

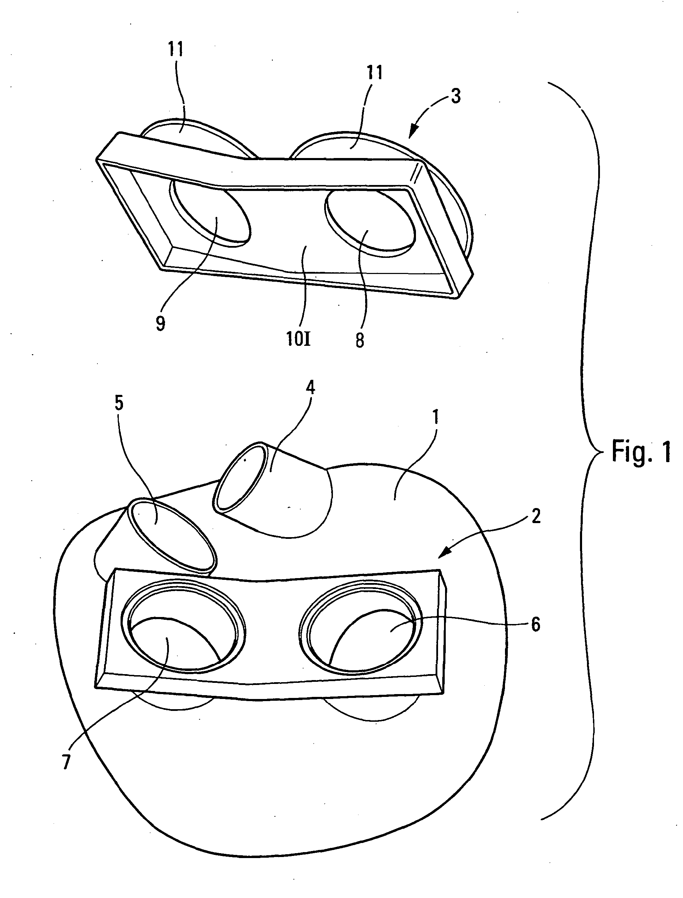

La prothèse cardiaque conforme à la présente invention, représentée sur la figure 1 de façon éclatée, est apte à remplacer les ventricules gauche et droit naturels d'un patient, après leur ablation. Elle comporte un corps rigide 1 dans lequel sont agencés des ventricules gauche et droit artificiels (non visibles sur la figure 1), lesdits ventricules étant pourvus de moyens de raccord 2, 3 aux oreillettes gauche et droite naturelles (non visibles) dudit patient, ainsi que de moyens de raccord 4 et 5, respectivement à l'artère pulmonaire et à l'aorte.

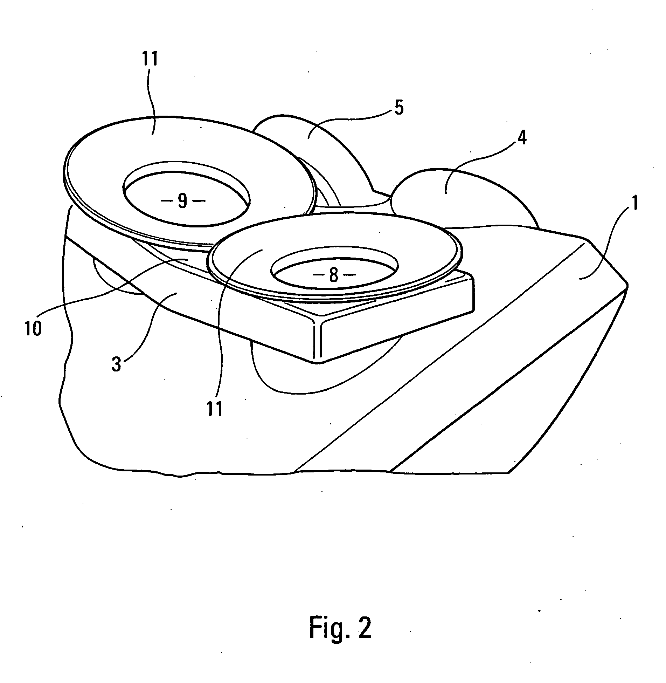

Les moyens de raccord auxdites oreillettes gauche et droite naturelles comportent :
- une lunette 2, faisant partie intégrante dudit corps rigide 1 et pourvue d'un orifice 6 en communication avec le ventricule artificiel droit et d'un orifice 7 en communication avec le ventricule artificiel gauche, des valves (non représentées sur la figure 1) étant disposées dans lesdits orifices 6, 7 ; et
- une lunette 3, sur laquelle peut être raccordée de façon amovible la lunette 2 et comportant, dans sa paroi 10, des orifices 8 et 9 destinés à être disposés respectivement en regard desdits orifices 6 et 7, lorsque lesdites lunettes occupent la position relative précise correspondant au fonctionnement de la prothèse (voir la figure 2).
Lesdites lunettes 2 et 3 présentent la forme générale de parallélépipèdes rectangles et la lunette 3 peut servir de couvercle à la lunette 2.

En position de fonctionnement de la prothèse, lorsque lesdites lunettes 2 et 3 sont emboîtées (voir la figure 2), celles-ci sont verrouillées en position par des moyens non représentés.

Comme on peut le voir sur les figures 1 et 2, chaque orifice 8, 9 est équipé d'une collerette individuelle 11, apte à permettre la suture de l'oreillette naturelle correspondante.

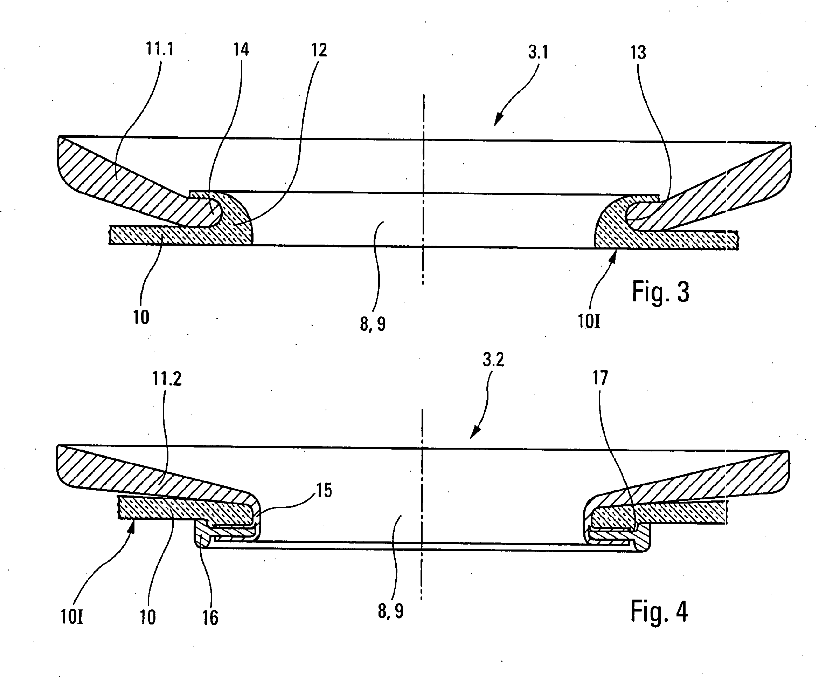

Dans le mode de réalisation 3.1 de la lunette 3, représenté sur la figure 3, est prévu, autour de chaque orifice 8, 9 et du côté opposé à la face interne 10I de la paroi 10, une saillie annulaire 12 pourvue d'une gorge périphérique 13. Le mode de réalisation 11.1 correspondant de la collerette individuelle 11 comporte une ouverture centrale délimitée par un bord périphérique interne 14, engagé dans ladite gorge périphérique 13.

On comprendra aisément que, dans l'exemple de réalisation de la figure 3, la collerette individuelle 11.1 peut aisément tourner autour de l'orifice 8, 9 correspondant, son bord périphérique interne 14 glissant alors en rotation dans ladite gorge périphérique 13. De plus, la collerette individuelle 11.1 peut aisément être montée et démontée de la saillie annulaire par déformation élastique dudit bord périphérique interne 14.

Dans le mode de réalisation 3.2 de la lunette 3, représenté sur la figure 4, les orifices 8 et 9 sont de simples trous pratiqués dans la paroi 10. En revanche, le mode de réalisation 11.2 de chaque collerette individuelle 11.2 comporte une ouverture centrale délimitée par un rebord 15 enserrant le bord de l'orifice 8, 9 correspondant et s'appliquant contre la face interne 10I de manière à favoriser l'hémocompatibilité en ne présentant au sang qu'une interface unique. De plus, le rebord 15 est prolongé périphériquement par un anneau de matière élastique 16, par exemple un élastomère, pouvant servir de joint d'étanchéité lorsqu'il est écrasé entre les lunettes 2 et 3, raccordées l'une à l'autre. Cet anneau 16 peut également participer au positionnement axial de la collerette 11.2 par rapport à l'orifice 8, 9 correspondant. A cet effet, sur ladite face interne 101, on peut prévoir une saillie annulaire 17, qui entoure lesdits orifices 8, 9 et derrière laquelle peut s'accrocher ledit anneau élastique 16.

Là encore, il est aisé de constater que les collerettes individuelles 11.2 peuvent tourner autour des orifices 8, 9 et peuvent y être montées et en être démontées par déformation élastique du rebord 15 et de l'anneau 16.

Les collerettes de suture individuelles 11, 11.1, 11.2 peuvent être réalisées en toute matière hémocompatible appropriée, par exemple multi-couche.

## Revendications

1. Prothèse cardiaque implantable dans la cavité péricardique d'un patient, ladite prothèse étant apte à remplacer les ventricules gauche et droit naturels dudit patient après ablation de ceux-ci et comportant un corps rigide (1) dans lequel sont agencés des ventricules artificiels gauche et droit, lesdits ventricules artificiels étant pourvus de moyens de raccord aux oreillettes gauche et droite naturelles dudit patient comportant :
- une première lunette (2), faisant partie intégrante dudit corps rigide (1) et comportant des premier et deuxième orifices (7, 6), respectivement en communication avec le ventricule artificiel gauche et avec le ventricule artificiel droit ; et
- une seconde lunette (3) comportant des troisième et quatrième orifices (8, 9) aptes à être respectivement reliés à ladite oreillette naturelle gauche et à ladite oreillette naturelle droite, lesdits troisième et quatrième orifices (8, 9) étant pourvus de moyens de jonction sur lesquels lesdites oreillettes naturelles sont respectivement suturées de façon étanche,
lesdites première et seconde lunettes (2, 3) étant aptes à être raccordées l'une à l'autre, de façon amovible, pour prendre une position de fonctionnement dans laquelle se trouvent respectivement en regard, d'une part, lesdits premier et troisième orifices (6, 8) et, d'autre part, lesdits deuxième et quatrième orifices (7, 9),
**caractérisée en ce que** lesdits moyens de jonction sont formés par des collerettes de suture individuelles (11) montées de façon à être libres en rotation, respectivement sur lesdits troisième et quatrième orifices (8, 9).

2. Prothèse cardiaque selon la revendication 1,
**caractérisée en ce que** lesdites collerettes de suture individuelles (11) sont montées de façon amovible sur lesdits troisième et quatrième orifices (8, 9), respectivement.

3. Prothèse cardiaque selon l'une des revendications 1 ou 2,
**caractérisée en ce que** chaque collerette de suture individuelle (11.1) comporte une ouverture centrale délimitée par un bord périphérique interne (14) et **en ce que** ladite seconde lunette (3) comporte, autour de chacun desdits troisième et quatrième orifices (8, 9), une saillie annulaire (12) formant une gorge (13) à l'intérieur de laquelle est engagé ledit bord (14) de ladite ouverture centrale.

4. Prothèse cardiaque selon l'une des revendications 1 ou 2,
**caractérisée en ce que** chaque collerette de suture individuelle (11.2) comporte une ouverture centrale délimitée par un rebord (15) apte à enserrer le bord de l'orifice (8, 9) correspondant.

5. Prothèse cardiaque selon la revendication 4,
**caractérisée en ce que** ledit rebord (15) de la collerette de suture (11.2) s'applique contre la face interne (101) de ladite seconde lunette (3) comprenant lesdits troisième et quatrième orifices (8, 9).

6. Prothèse cardiaque selon la revendication 5,
**caractérisé en ce que** ledit rebord (15) est prolongé périphériquement par un anneau de matière élastique (16) coopérant avec ladite face interne (10I) pour assurer le positionnement axial de ladite collerette par rapport à l'orifice (8, 9) correspondant.

7. Prothèse cardiaque selon la revendication 6,
**caractérisée en ce que** la face interne (10I) de ladite seconde lunette (3) comporte une saillie annulaire (17), qui entoure ledit troisième ou quatrième orifice (8, 9) correspondant et derrière laquelle peut s'accrocher élastiquement ledit anneau élastique (16).

## Claims

1. Heart prosthesis implantable in the pericardial cavity of a patient, said prosthesis being able to replace the natural left and right ventricles of said patient after their removal, and comprising a rigid body (1) in which artificial left and right ventricles are arranged, said artificial ventricles being provided with means for connection to the natural left and right auricles of said patient, comprising:
- a first bezel (2) forming an integral part of said rigid body (1) and comprising first and second orifices (7, 6), which communicate respectively with the artificial left ventricle and with the artificial right ventricle; and
- a second bezel (3) comprising third and fourth orifices (8, 9) that can be connected respectively to said natural left auricle and to said natural right auricle, said third and fourth orifices (8, 9) being provided with joining means on which said natural auricles are respectively sutured in a leaktight manner,
said first and second bezels (2, 3) being able to be connected to each other removably, in order to assume an operative position in which said first and third orifices (6, 8) are located opposite each other and said second and fourth orifices (7, 9) are located opposite each other,
**characterized in that** said joining means are formed by individual suture flanges (11) mounted in such a way as to be free in rotation on said third and fourth orifices (8, 9), respectively.

2. Heart prosthesis according to Claim 1, **characterized in that** said individual suture flanges (11) are mounted removably on said third and fourth orifices (8, 9), respectively.

3. Heart prosthesis according to one of Claims 1 and 2, **characterized in that** each individual suture flange (11.1) comprises a central opening delimited by an inner peripheral edge (14), and **in that** said second bezel (3) comprises, around each of said third and fourth orifices (8, 9), an annular projection (12) forming a groove (13) inside of which said edge (14) of said central opening is engaged.

4. Heart prosthesis according to one of Claims 1 and 2, **characterized in that** each individual suture flange (11.2) comprises a central opening delimited by a rim (15) that can enclose the edge of the corresponding orifice (8, 9).

5. Heart prosthesis according to Claim 4, **characterized in that** said rim (15) of the suture flange (11.2) bears against the inner face (10I) of said second bezel (3) comprising said third and fourth orifices (8, 9).

6. Heart prosthesis according to Claim 5, **characterized in that** said rim (15) is continued peripherally by a ring (16) of elastic material that cooperates with said inner face (10I) in order to ensure the axial positioning of said flange with respect to the corresponding orifice (8, 9).

7. Heart prosthesis according to Claim 6, **characterized in that** the inner face (10I) of said second bezel (3) comprises an annular projection (17) which surrounds said third or fourth orifice (8, 9) and behind which said elastic ring (16) can fasten itself elastically.

## Patentansprüche

1. Herzprothese, welche in die Perikardhöhle eines Patienten implantierbar ist, wobei die Prothese geeignet ist, die natürliche linke und rechte Herzkammer des Patienten nach Ablation dieser zu ersetzen, und wobei sie einen starren Körper (1) umfasst, in dem eine künstliche linke und rechte Herzkammer gebildet ist, wobei die künstlichen Herzkammern mit Mitteln zum Anschluss an das natürliche linke und rechte Herzohr des Patienten versehen sind, umfassend:
- eine erste Brille (2), die integraler Bestandteil des starren Körpers (1) ist und eine erste und eine zweite Öffnung (7, 6) umfasst, die mit der künstlichen linken Herzkammer bzw. mit der künstlichen rechten Herzkammer in Verbindung stehen; und
- eine zweite Brille (3), die eine dritte und eine vierte Öffnung (8, 9) umfasst, die geeignet sind, mit dem natürlichen linken Herzohr bzw. dem natürlichen rechten Herzohr verbunden zu werden, wobei die dritte und die vierte Öffnung (8, 9) mit Verbindungsmitteln versehen sind, an die die natürlichen Herzohren jeweils auf dichte Weise genäht sind,
wobei die erste und die zweite Brille (2, 3) geeignet sind, auf lösbare Weise miteinander verbunden zu werden, um eine Betriebsposition einzunehmen, in der einerseits die erste und die dritte Öffnung (6, 8) bzw. andererseits die zweite und die vierte Öffnung (7, 9) einander gegenüberliegen,
**dadurch gekennzeichnet, dass** die Verbindungsmittel durch individuelle Nahtflansche (11) gebildet werden, die auf drehbewegliche Weise an der dritten bzw. vierten Öffnung (8, 9) montiert sind.

2. Herzprothese nach Anspruch 1,
**dadurch gekennzeichnet, dass** die individuellen Nahtflansche (11) auf lösbare Weise an der dritten bzw. vierten Öffnung (8, 9) montiert sind.

3. Herzprothese nach einem der Ansprüche 1 oder 2,
**dadurch gekennzeichnet, dass** jeder individuelle Nahtflansch (11.1) eine mittige Öffnung umfasst, die durch einen inneren Umfangsrand (14) begrenzt ist, und dass die zweite Brille (3) um die dritte und die vierte Öffnung (8, 9) jeweils einen ringförmigen Vorsprung (12) umfasst, der eine Rille (13) bildet, in die der Rand (14) der mittigen Öffnung eingefügt ist.

4. Herzprothese nach einem der Ansprüche 1 oder 2, **dadurch gekennzeichnet, dass** jeder individuelle Nahtflansch (11.2) eine mittige Öffnung umfasst, die durch eine Umstülpung (15) begrenzt wird, die geeignet ist, den Rand der entsprechenden Öffnung (8, 9) zu umschließen.

5. Herzprothese nach Anspruch 4,
**dadurch gekennzeichnet, dass** die Umstülpung (15) des Nahtflansches (11.2) an der Innenseite (10I) der zweiten Brille (3) anliegt, die die dritte und die vierte Öffnung (8, 9) umfasst.

6. Herzprothese nach Anspruch 5,
**dadurch gekennzeichnet, dass** die Umstülpung (15) am Umfang durch einen Ring aus elastischem Material (16) verlängert wird, der mit der Innenseite (10I) zusammenarbeitet, um die axiale Positionierung des Flansches in Bezug auf die entsprechende Öffnung (8, 9) zu gewährleisten.

7. Herzprothese nach Anspruch 6,
**dadurch gekennzeichnet, dass** die Innenseite (10I) der zweiten Brille (3) einen ringförmigen Vorsprung (17) umfasst, der die entsprechende dritte oder vierte Öffnung (8, 9) umgibt und hinter dem der elastische Ring (16) auf elastische Weise eingehängt werden kann.
